# EUROPEAN PATENT SPECIFICATION

(11) **EP 3 024 419 B1**
(45) Date of publication and mention of the grant of the patent: **11.05.2022**
(21) Application number: 14829809.4
(22) Date of filing: 24.07.2014
(51) Int. Cl.: A61F 2/44, A61B 17/86, A61F 2/30, A61F 2/34, B22F 3/105, B22F 5/00

(54) **SURGICAL IMPLANT DEVICES INCORPORATING POROUS SURFACES**
CHIRURGISCHE IMPLANTATVORRICHTUNGEN MIT PORÖSEN OBERFLÄCHEN
DISPOSITIFS POUR IMPLANT CHIRURGICAL INCORPORANT DES SURFACES POREUSES

(30) Priority: 24.07.2013 US 201361857824 P; 02.10.2013 US 201361885778 P; 16.12.2013 US 201361916469 P
(43) Date of publication of application: 01.06.2016
(73) Proprietor: Kyocera Medical Technologies, Inc., Redlands, CA 92374 (US)
(72) Inventor: STEINMANN, John, C., Redlands, CA 92373 (US); RUCKER, Scott, Austin, Texas 78727 (US); RASMUSSEN, Tim, Redlands, California 92374 (US); STEINMANN, John, P., Redlands, CA 92374 (US); CAWLEY, Trace, Austin, Texas 78732 (US); ROSS, Thomas, Austin, TX 78759 (US); RIOS, Ernesto, Austin, TX 78759 (US); OLCESE, Andrew, Austin, TX 78759 (US)
(74) Representative: dompatent von Kreisler Selting Werner - Partnerschaft von Patent- und Rechtsanwälten mbB
(86) International application number: PCT/US2014/047940
(87) International publication number: WO 2015/013479

(56) References cited:
- US-A- 4 272 855
- US-A1- 2007 225 785
- US-A1- 2008 109 081
- US-A1- 2008 206 297
- US-A1- 2009 105 831
- US-A1- 2009 326 671
- US-A1- 2010 298 950
- US-A1- 2010 298 950
- US-A1- 2011 035 010
- US-A1- 2013 056 912
- US-A1- 2013 056 912

## Description

### FIELD OF THE INVENTION

The present invention relates generally to novel surgical implant devices incorporating porous surfaces. More specifically, the present invention relates to novel surgical implant devices incorporating porous surfaces for enhancing bony fixation and purchase when implanted. These porous surfaces are formed via novel additive manufacturing techniques and, optionally, novel post-processing manufacturing techniques. They include novel needle-like protrusions and lattice structures.

### BACKGROUND OF THE INVENTION

When various surgical implant devices, well known to those of ordinary skill in the art, are placed adjacent to or between bony surfaces, it is desirable that adequate friction is present to hold them in place and that surfaces are available for bony fixation and purchase over time. Accordingly, these surgical implant devices often incorporate mechanically-manufactured friction surfaces or utilize friction coatings or bondings for such purposes. However, these mechanically-manufactured friction surfaces, typically consisting of teeth, grooves, striations, or the like, are often not adequate and do little to promote bony purchase. Similarly, these friction coatings or bondings may delaminate and fail.

US 2010/298950 A1 discloses a bone implant comprising a core having an integral anchoring structure at its surface. The structure comprises an array of upwardly extending mutually spaced pointed claw-like teeth for digging into bone to which the implant is to be attached, and a network of pores underlying the array and communicating with the exterior of the surface via openings between the teeth, the pores allowing for the circulation of nutrients to promote bone growth.

US 2013/056912 A1 describes a method of producing a three-dimensional porous tissue in-growth structure. The method includes the steps of depositing a first layer of metal powder and scanning the first layer of metal powder with a laser beam to form a portion of a plurality of predetermined unit cells. Depositing at least one additional layer of metal powder onto a previous layer and repeating the step of scanning a laser beam for at least one of the additional layers in order to continuing forming the predetermined unit cells. The method further includes continuing the depositing and scanning steps to form a medical implant. US 2011/153025 A1, WO 2009/034429 A2 and WO 2007/113862 A1 describe further examples of meshed or latticed implants and method for manufacturing the latter with various additive manufacturing techniques.

US 2008/109081 A1 provides articulating devices for replacing damaged or degenerated weight bearing joints. The devices may have two or more surfaces that articulate against one another that are coated or fully formed of PEEK or similar materials to provide improved wear capabilities while maintaining sufficient strength to operate in a weight bearing capacity.

US 2008/206297 A1 describes synthetic composite materials for use, for example, as orthopedic implants. In one example, a composite material for use as a scaffold includes a thermoplastic polymer forming a porous matrix that has continuous porosity and a plurality of pores. The porosity and the size of the pores are selectively formed during synthesis of the composite material. The example composite material also includes a plurality of a anisometric calcium phosphate particles integrally formed, embedded in, or exposed on a surface of the porous matrix. The calcium phosphate particles provide one or more of reinforcement, bioactivity, or bioresorption.

US 2009/105831 A1 provides a method and apparatus for use with a surgical implant to secure fasteners, such as bone screws or pins. An anti-backout mechanism is used to preventing migration of the fasteners after the implant is installed. In one example, an anti-backout mechanism includes a locking plate having a locked position and an unlocked position. The locking plate has one or more protrusions that are configured to prevent migration the fasteners when the locking plate is in the locked position.

US 2011/035010 A1 discloses an intervertebral implant for insertion between adjacent vertebral bodies. The intervertebral implant can include a first component including a first articulating surface, which can be generally convex with a first radius of curvature. The first articulating surface can also define a stop element. The intervertebral implant can comprise a second component defining an aperture and including a second articulating surface. The second articulating surface can be generally concave and can have a second radius of curvature. The second articulating surface can be articulable with the first articulating surface for retaining motion between the first and second vertebra. A portion of the first articulating surface including the stop element can extend into the aperture and contacts a sidewall of the aperture to limit the range of articulation of the first component and the second component.

Thus, what are still needed in the art are improved surgical implant devices that incorporate essentially-integral friction surfaces that are strong and durable, and that provide adequate surface area for bony fixation and purchase, while still being economical to manufacture. Improved additive and post-processing manufacturing techniques now make this possible.

### BRIEF SUMMARY OF THE INVENTION

The present invention relates to an surgical implant device according to claim 1. Preferred embodiments of the invention are set forth in the dependent claims.

In one exemplary embodiment, the present invention provides a surgical implant device, comprising: a metallic body portion; and one or more metallic surfaces comprising a metallic osteo-porous or osteo-derived layer and a plurality of needles on an exterior portion of the metallic body portion; wherein the metallic body portion and the one or more metallic surfaces comprising the metallic osteo-porous or osteo-derived layer and the plurality of needles are integrally formed.

According to the invention, the metallic osteo-porous or osteo-derived layer and the plurality of needles are integrally formed, wherein the metallic osteo-porous or osteo-derived layer comprises an irregular lattice of intersecting struts that intersect at randomly perturbed nodes. According to the invention, some of the plurality of needles are anchored to the metallic body portion and some of the plurality of needles are anchored to the intersecting struts of the irregular lattice of the metallic osteo-porous or osteo-derived layer, wherein the plurality of needles substantially increase the coefficient of friction of the one or more metallic surfaces.

The one or more surfaces comprising the plurality of needles may be formed by an additive manufacturing process. Optionally, the plurality of needles are disposed substantially perpendicular to the body portion. Alternatively, the plurality of needles are disposed at an angle to the body portion. Preferably, the plurality of needles comprise titanium. The body portion defines a hollow interior cavity. Optionally, the body portion defines one or more ports that are configured to receive a bone screw. The body portion defines one or more ports that are configured to allow bony ingrowth. The surgical implant device comprises one of an anterior lumbar interbody fusion cage, a posterior lumbar interbody fusion cage, a transforaminal lumbar interbody fusion cage, an oblique lumbar interbody fusion cage, a cervical cage, and a bone screw.

The present disclosure further describes a method for manufacturing a surgical implant device, comprising: providing a metallic body portion; and forming one or more metallic surfaces comprising a metallic osteo-porous or osteo-derived layer and a plurality of needles on an exterior portion of the metallic body portion, ; wherein the metallic body portion and the one or more metallic surfaces comprising the metallic osteo-porous or osteo-derived layer and the plurality of needles are integrally formed; wherein the metallic osteo-porous or osteo-derived layer comprises an irregular lattice of intersecting struts that intersect at randomly perturbed nodes. According to the invention, some of the plurality of needles are anchored to the metallic body portion and some of the plurality of needles are anchored to the intersecting struts of the irregular lattice of the metallic osteo-porous or osteo-derived layer, wherein the plurality of needles substantially increase the coefficient of friction of the one or more metallic surfaces.

The one or more surfaces comprising the plurality of needles may be formed by an additive manufacturing process. Optionally, the plurality of needles are disposed substantially perpendicular to the body portion. Alternatively, plurality of needles are disposed at an angle to the body portion. Preferably, the plurality of needles comprise titanium. The body portion defines a hollow interior cavity. Optionally, the body portion defines one or more ports that are configured to receive a bone screw. The body portion defines one or more ports that are configured to allow bony ingrowth.

### BRIEF DESCRIPTION OF THE DRAWINGS

The present invention is illustrated and described herein with reference to the various drawings, in which like reference numbers are used to denote like device components/method steps, as appropriate, and in which:

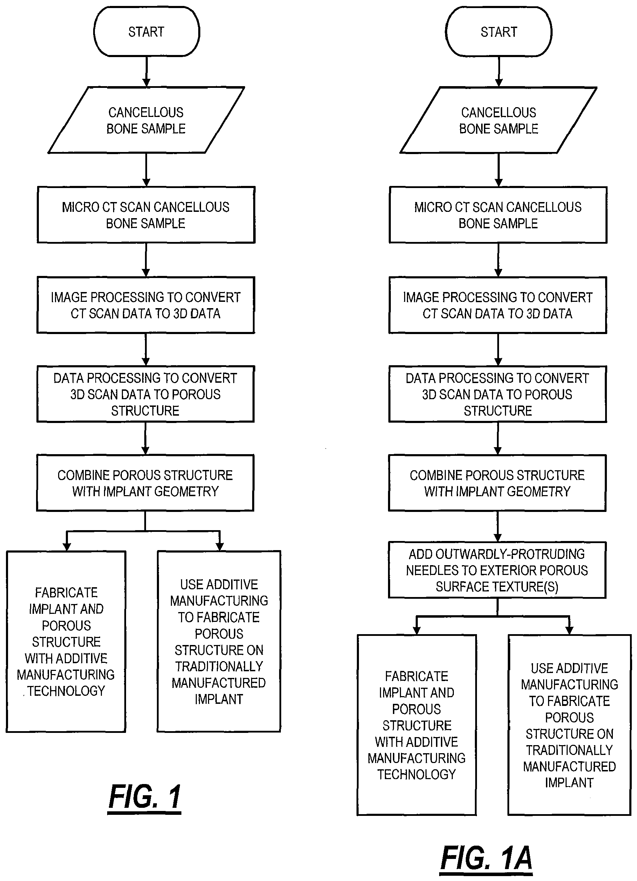

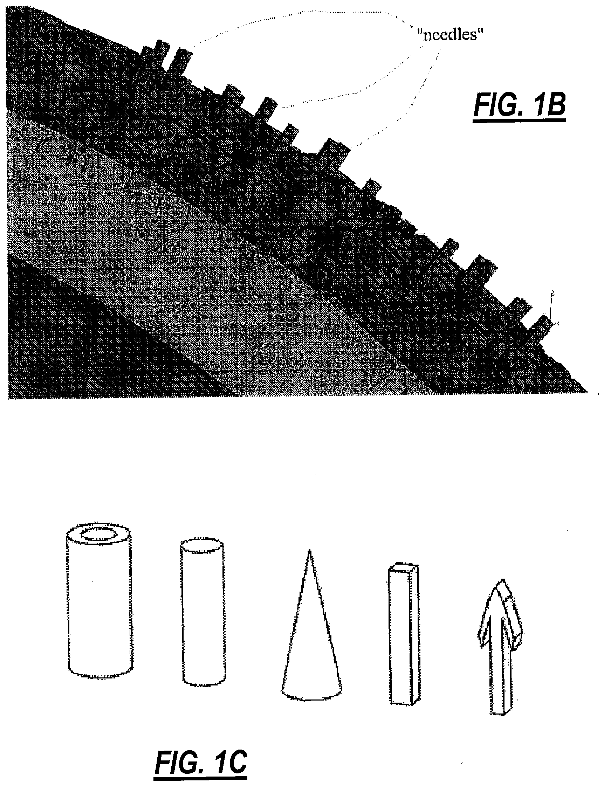

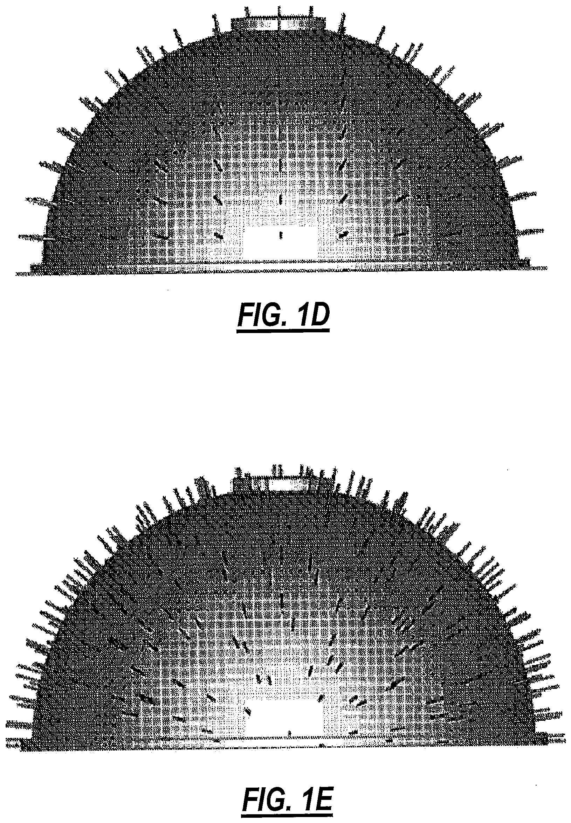

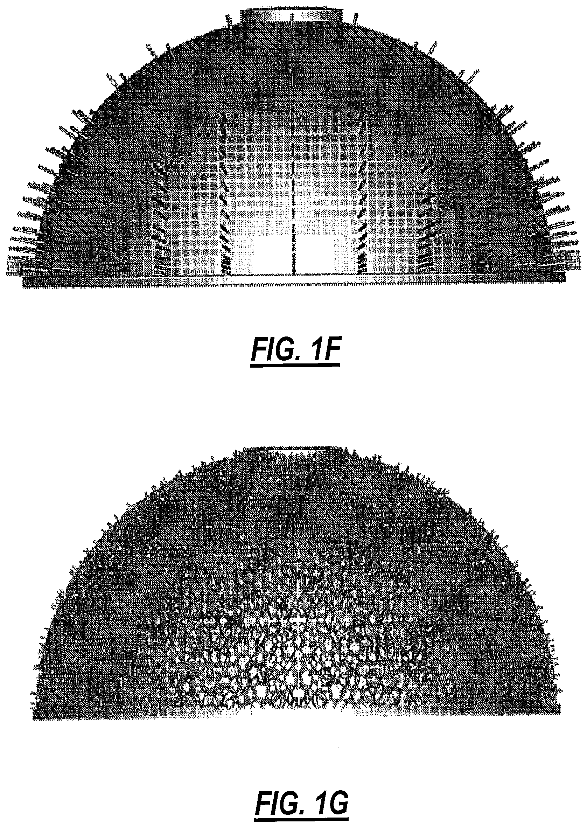

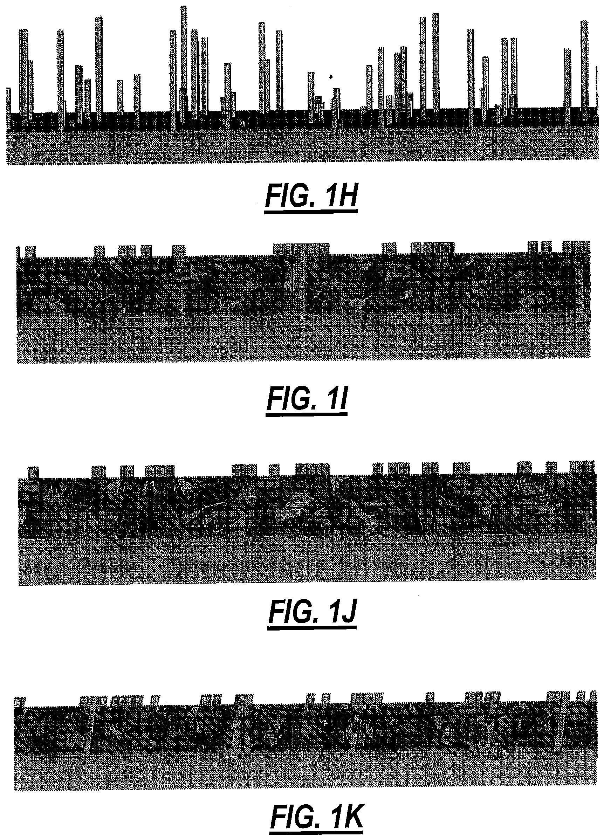

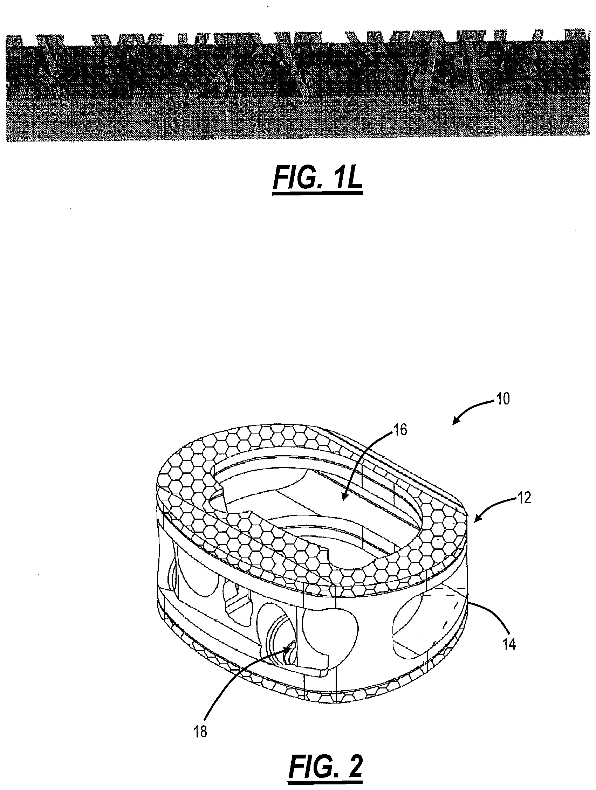

FIG. 1 depicts an exemplary method for producing surgical implant devices having osteo-derived and/or osteo-porous surface(s).
FIG. 1A depicts an exemplary method for producing surgical implant devices having osteo-derived and/or osteo-porous surface(s) with additional outwardly-protruding "needles."
FIG. 1B depicts an exploded section of a computer-generated rendering of an exemplary osteo-derived and/or osteo-porous surface with outwardly-protruding needles.
FIG. 1C conceptually depicts various exemplary shapes of outwardly-protruding needles (including, from left to right, tubular, rod-shaped, conical, square/rectangular, and barbed/irregular) useful with the present invention.
FIG. ID conceptually depicts an exemplary surgical implant device surface containing a uniform distribution of outwardly-protruding needles.
FIG. IE conceptually depicts an exemplary surgical implant device surface containing a nonuniform distribution of outwardly-protruding needles.
FIG. IF conceptually depicts an exemplary surgical implant device surface containing a gradient distribution of outwardly-protruding needles.
FIG. 1G conceptually depicts an exemplary osteo-porous, osteo-derived, and/or trabecular surgical implant device surface containing a randomized distribution of outwardly-protruding needles.
FIG. 1H conceptually depicts an exemplary cross-section of an implant surface containing a randomized distribution of needle heights.
FIG. 1I conceptually depicts an exemplary cross-section of an implant surface containing an exemplary osteo-porous, osteo-derived, and/or trabecular coating with needles anchored to the underlying substrate.
FIG. 1J conceptually depicts an exemplary cross-section of an implant surface containing an exemplary osteo-porous, osteo-derived, and/or trabecular coating with needles anchored to the osteo-porous, osteo-derived, and/or trabecular coating.
FIG. IK conceptually depicts an exemplary cross-section of an implant surface containing an exemplary osteo-porous, osteo-derived, and/or trabecular coating with uniformly tilted needles anchored to the underlying substrate.
FIG. 1L conceptually depicts an exemplary cross-section of an implant surface containing an exemplary osteo-porous, osteo-derived, and/or trabecular coating with non-uniformly tilted needles anchored to the underlying substrate.
FIG. 2 is a perspective view of one exemplary embodiment of an anterior lumbar interbody fusion (ALIF) cage of the present invention, again highlighting the porous surfaces thereof formed by the techniques of FIG. 1.
FIG. 3 is a series of perspective and planar views of one exemplary embodiment of the ALIF cage of the present invention, again highlighting the porous surfaces thereof formed by the techniques of FIG. 1.
FIG. 4 is a photograph of one exemplary embodiment of the ALIF cage of the present invention, again highlighting the porous surfaces thereof formed by the techniques of FIG. 1.
FIG. 5 is another photograph of one exemplary embodiment of the ALIF cage of the present invention, again highlighting the porous surfaces thereof formed by the techniques of FIG. 1.
FIG. 6 depicts preferred process flows for manufacturing abrasive-blasted implants in accordance with the invention.
FIG. 7 depicts alternative process flows for manufacturing abrasive-blasted implants in accordance with the invention.

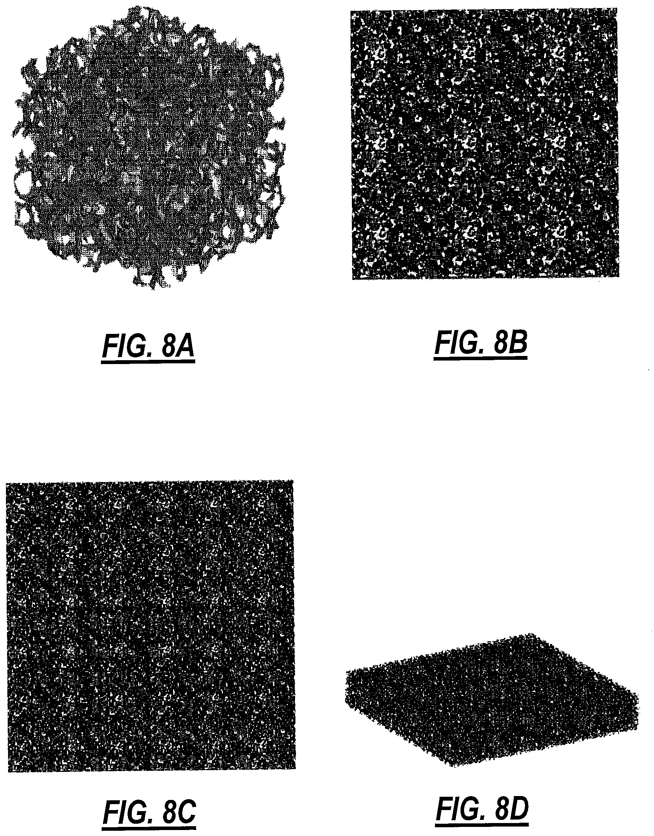

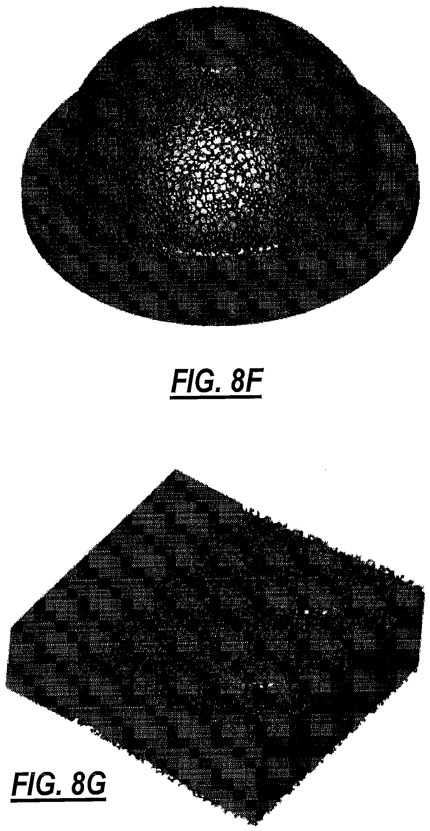

FIG. 8A depicts an illustrative, irregular (e.g., trabecular, osteo-porous, and/or osteo-derived) unit cell appropriate for additive manufacture in accordance with the invention.
FIG. 8B shows the unit cell of FIG. 8A repeated in a 3x3 array.
FIG. 8C shows the unit cell of FIG. 8A repeated in a 6x6 array.
FIG. 8D shows an isometric view of a 9x9 array of the unit cell of FIG. 8A.
FIG. 8E shows the unit cell of FIG. 8A patterned across the surface of an acetabular shell.
FIG. 8F shows the unit cell of FIG. 8A patterned across the surface of an ALIF cage "blank."
FIG. 9A conceptually depicts a 2-D rendering of a cubic lattice structure, with no node perturbation (not claimed) and no strut size randomization.
FIG. 9B conceptually depicts a 3-D rendering of the cubic lattice structure, with no node perturbation (not claimed) and no strut size randomization.
FIG. 9C conceptually depicts a 2-D rendering of a cubic lattice structure, with 20% node perturbation and no strut size randomization.
FIG. 9D conceptually depicts a 3-D rendering of the cubic lattice structure, with 20% node perturbation and no strut size randomization.
FIG. 9E conceptually depicts a 2-D rendering of a cubic lattice structure, with 40% node perturbation and no strut size randomization.
FIG. 9F conceptually depicts a 3-D rendering of the cubic lattice structure, with 40% node perturbation and no strut size randomization.
FIG. 9G conceptually depicts a 2-D rendering of a cubic lattice structure, with 20% node perturbation and 100% strut size randomization.
FIG. 9H conceptually depicts a 3-D rendering of the cubic lattice structure, with 20% node perturbation and 100% strut size randomization.
FIG. 91 conceptually depicts a 2-D rendering of a dodecahedron lattice structure, with no node perturbation (not claimed) and no strut size randomization.

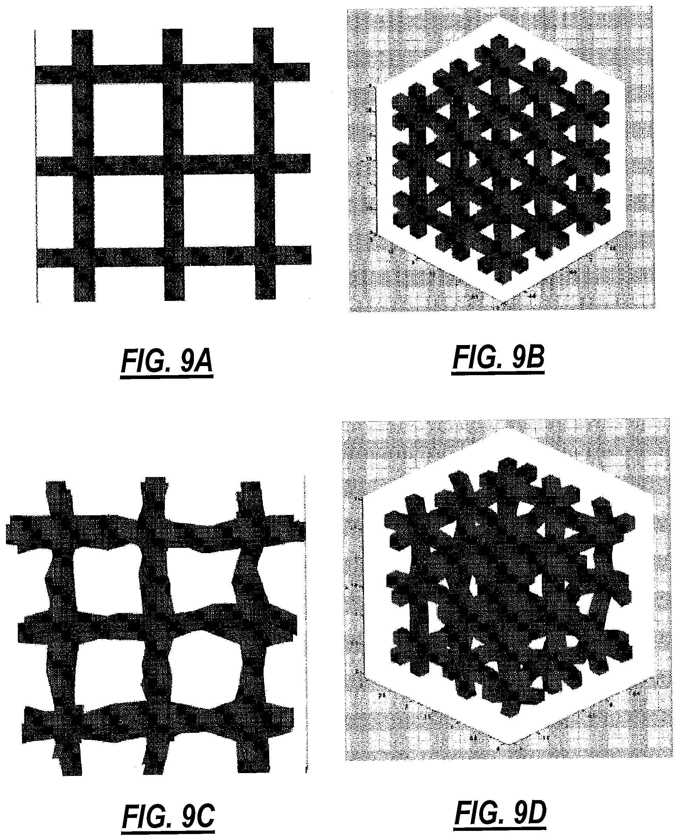

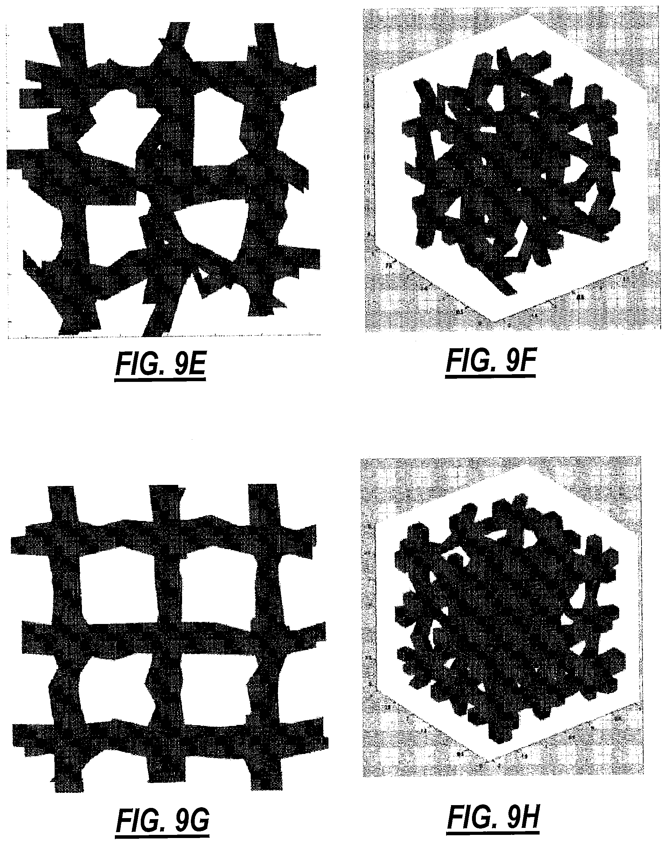

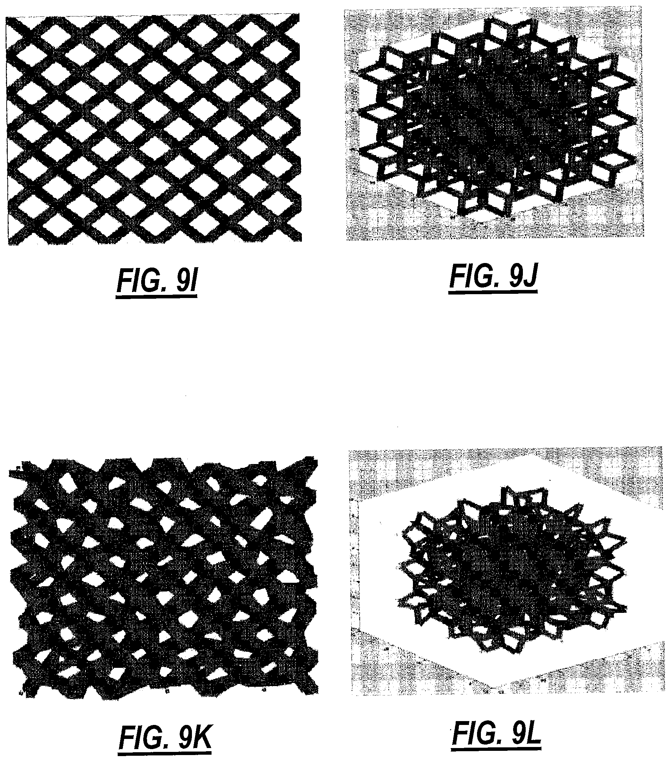

FIG. 9J conceptually depicts a 3-D rendering of the dodecahedron lattice structure, with no node
perturbation (not claimed) and no strut size randomization.
FIG. 9K conceptually depicts a 2-D rendering of a dodecahedron lattice structure, with 20% node perturbation and no strut size randomization.
FIG. 9L conceptually depicts a 3-D rendering of the dodecahedron lattice structure, with 20% node perturbation and no strut size randomization.

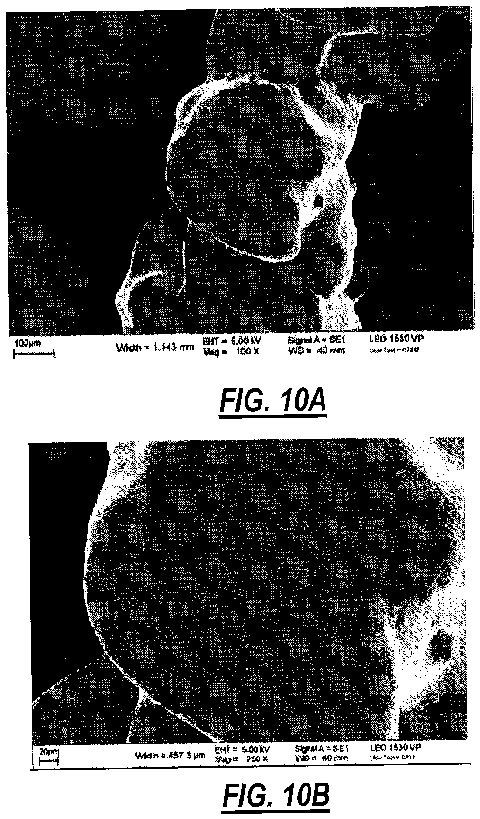

FIGS. 10A and 10B depict exemplary SEM views of porous/trabecular metal sections fabricated by additive manufacturing in accordance with the invention, but not subjected to a post-fabrication abrasive blast step.

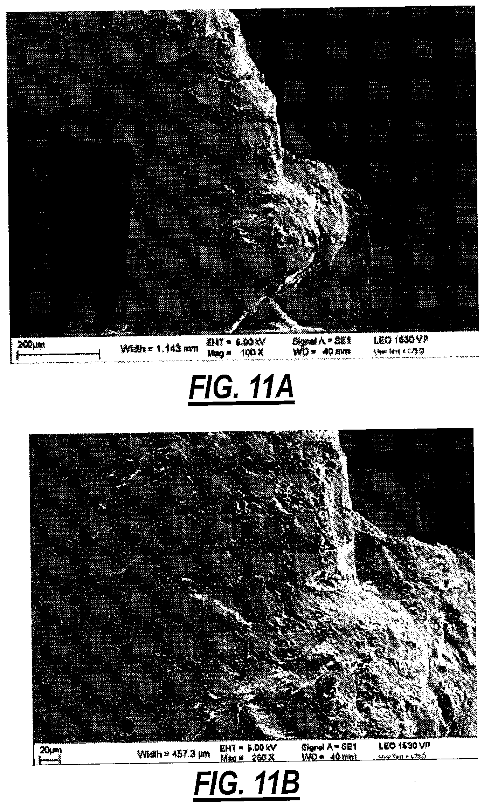

FIGS. 11 A and 11B depict exemplary SEM views of porous/trabecular metal sections fabricated by additive manufacturing in accordance with the invention, after being subjected to a post-fabrication abrasive blast step.

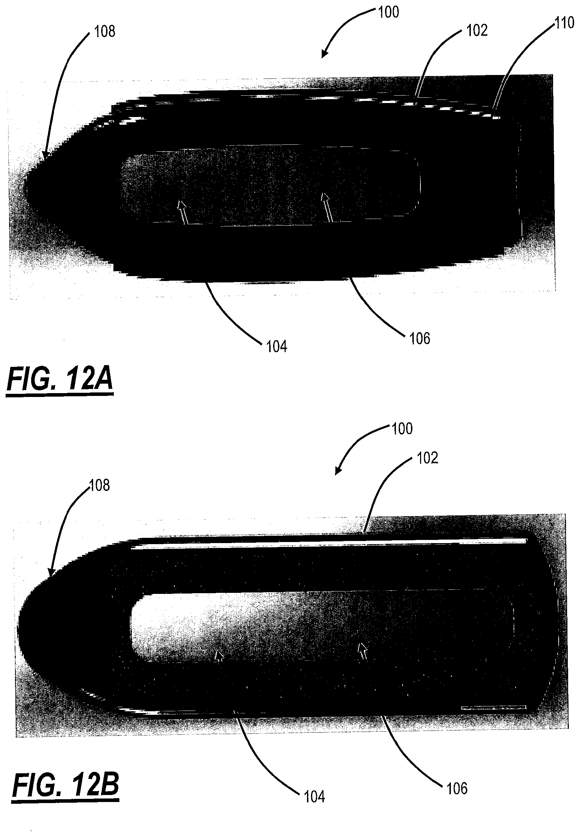

FIGS. 12A and 12B are side and top planar views of one exemplary embodiment of the posterior lumbar interbody fusion (PLIF) cage of the present invention.

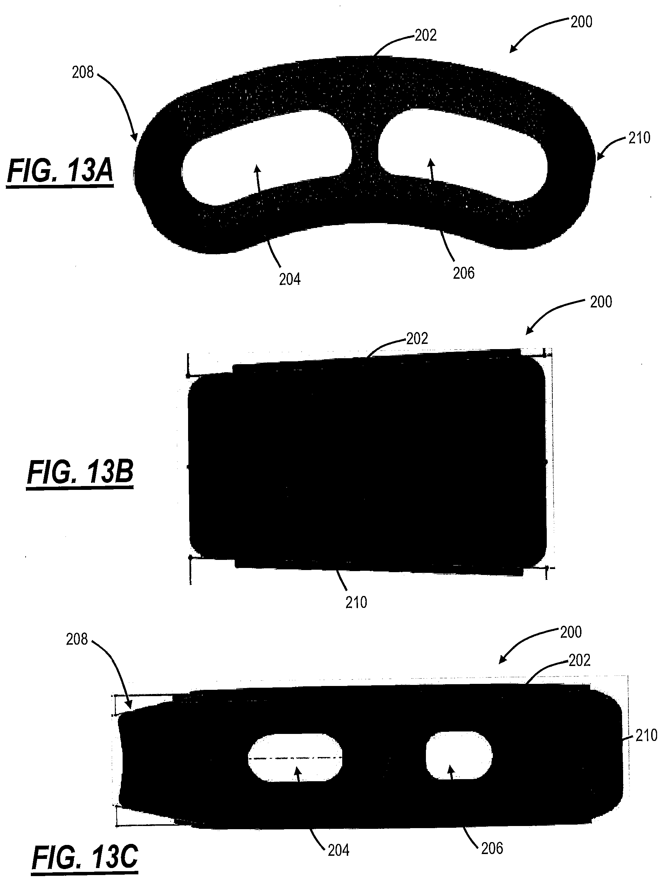

FIGS. 13A-13C are top planar, side planar, and bullet views of one exemplary embodiment of the transforaminal lumbar interbody fusion (TLIF) cage of the present invention.

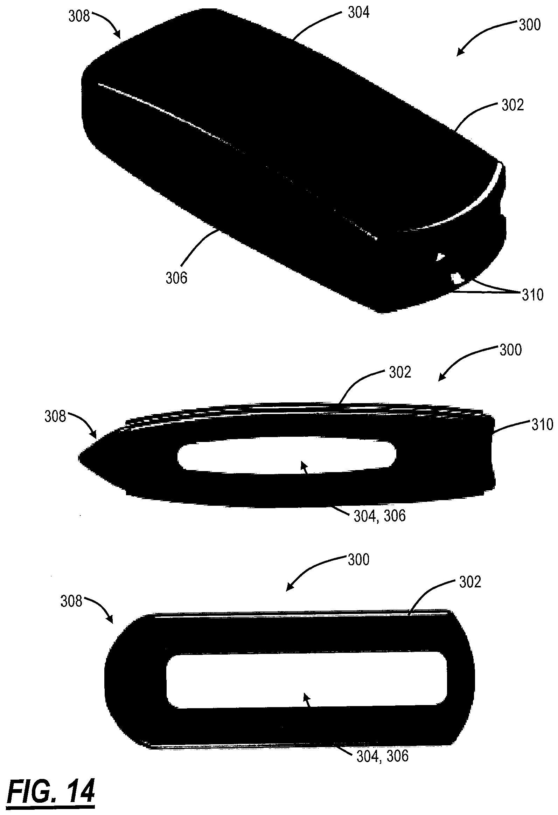

FIG. 14 is perspective, side planar, and top planar views of one exemplary embodiment of the oblique lumbar interbody fusion (OLIF) cage of the present invention.

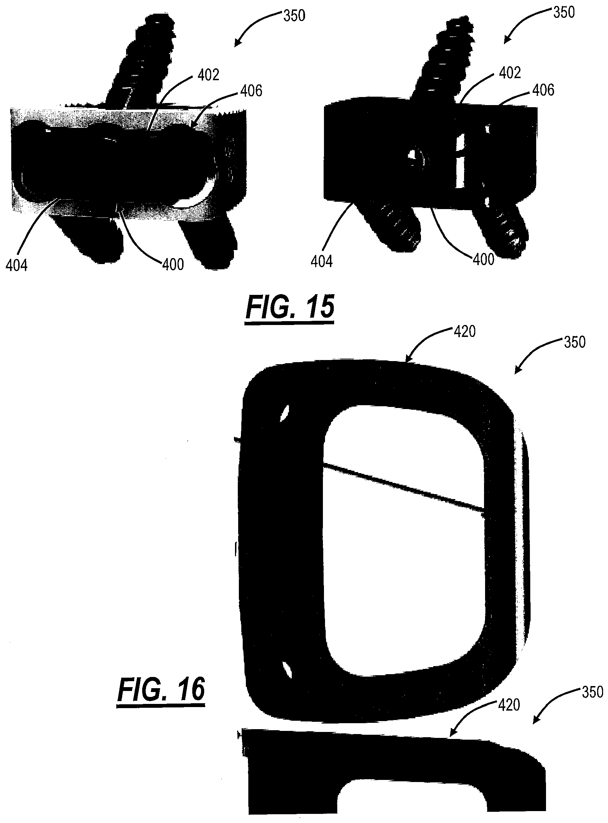

FIG. 15 is perspective views of the cervical cages of the present invention, both in PEEK and titanium embodiments, and incorporating a novel locking mechanism.
FIG. 16 is top and side planar views of the cervical cage of the present invention, in the titanium embodiment and including at least one porous surface formed by the process of FIG. 1.

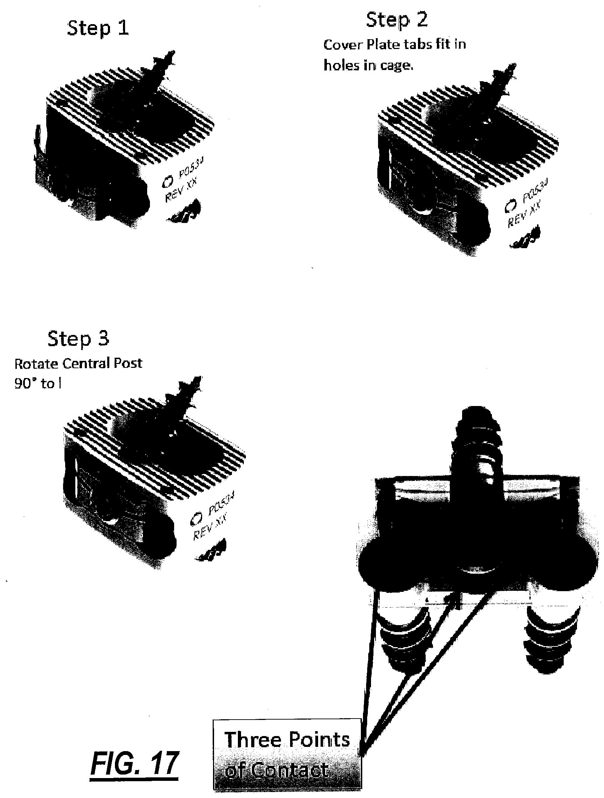

FIG. 17 is a series of perspective views illustrating the operation of the locking mechanism of FIG. 15.

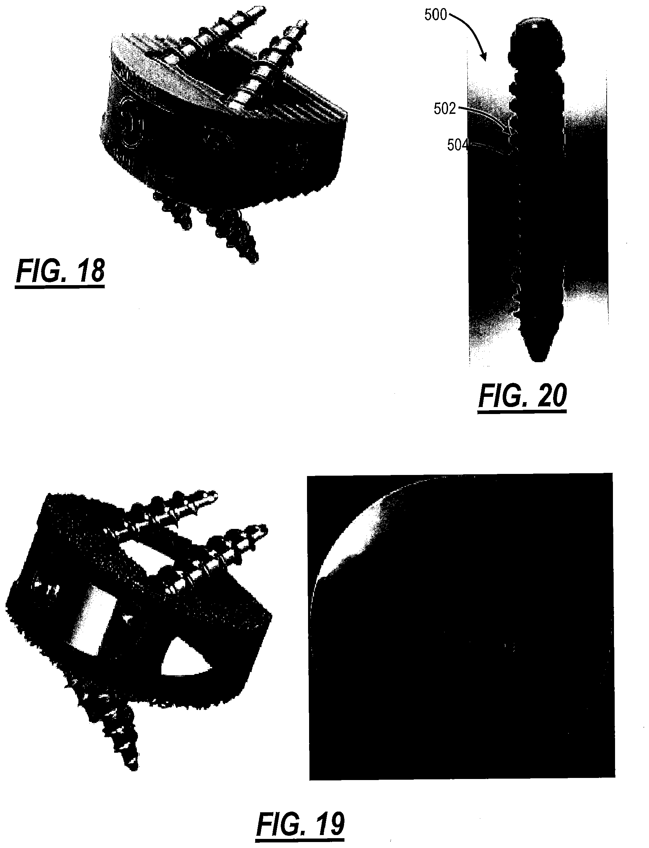

FIG. 18 is a perspective view of the ALIF cage of the present invention, in a PEEK embodiment and including a novel locking mechanism.
FIG. 19 is a perspective view of the ALIF cage of the present invention, in a PEEK embodiment and including a novel locking mechanism.
FIG. 20 is a perspective view of the surgical screw of the present invention, incorporating both non-porous and porous regions.

### DETAILED DESCRIPTION OF THE INVENTION

Generally speaking, and without intending to be limiting, one aspect of the invention relates to improved medical implants that include, for example, at least the following: a primary structure formed from metal; and at least one needle-populated, metallic surface portion formed on at least one exterior portion of the primary structure, the at least one surface portion located such that it engages with a patient's bone when the implant is implanted in the patient. Such needle-populated, metallic surface portions may contain, for example, a collection of at least fifty, a hundred, two hundred, five-hundred or more needles, and may be further characterized by at least one, two, three, four, five or more of the following characteristics: (a) the needles in the collection are all oriented substantially normal to the surface portion; (b) the needles in the collection are all oriented in substantially the same direction, with the direction being other than normal to the surface portion; (c) the needles in the collection are all oriented in substantially the same direction, with the direction being other than normal to the surface portion, but within 15 degrees from the normal direction; (d) the needles in the collection are all oriented in substantially the same direction, with the direction being other than normal to the surface portion, and more than 15 degrees from the normal direction; direction (not claimed); (e) the collection includes needles oriented in at least three different directions relative to the surface portion; (f) the collection includes needles oriented in at least five different directions relative to the surface portion, with all of the needles oriented within 15 degrees from the surface portion normal direction; (g) all of the needles in the collection have substantially the same height; (h) the collection includes needles of at least three different heights; (i) all of the needles in the collection have substantially the same shape; (j) the collection includes needles of at least two different shapes; (k) the needles are distributed substantially uniformly over the surface portion; (1) the needles are distributed non-uniformly over the surface portion; (m) all of the needles in the collection are anchored to the primary structure; (n) most of the needles in the collection are anchored to the primary structure; (o) most of the needles in the collection are anchored to structural elements contained within an osteo-porous, osteo-derived or trabecular coating on the at least one exterior portion of the primary structure; and/or (p) all of the needles in the collection are anchored to structural elements contained within an osteo-porous, osteo-derived or trabecular coating on the at least one exterior portion of the primary structure. The at least one exterior portion preferably includes at least one osteo-porous surface, which may comprise at least one osteo-derived surface. The at least one osteo-porous surface and the needles may be simultaneously formed by an additive manufacturing process, such as, but not limited to, EBM or DMSLS. The primary structure may comprise, for example, a dental implant, a foot-and-ankle or long-bone osteotomy wedge, an intervertebral fusion device, a tibial/femoral augment or spacer, a tibial tray portion of a knee implant, a femoral component portion of a knee implant, a primary hip implant, a revision hip implant, a hip trauma component, an acetabular cup, a hip acetabular augment, or other appropriate structure.

Again, generally speaking, and without intending to be limiting, another aspect of the invention relates to method(s) for making a medical implants with at least one osteo-porous surface by, for example: forming at least a portion of a primary structure of the implant; and forming at least one needle-populated, metallic surface portion on at least one exterior portion of the primary structure using an additive manufacturing technique, the at least one needle-populated surface portion located such that it engages with a patient's bone when the implant is implanted in the patient.

Other aspects of the invention relate to additional features, structures, processes and materials depicted in the figures and/or described herein.

Referring to FIG. 1, the exemplary flow starts with a spongy bone sample, which is micro CT scanned to obtain 3D scan data, which is then processed into solid model data representing an osteo-porous or osteo-derived texture. This texture data is then combined with data representing the overall implant geometry to create a fabrication file for use by either of the manufacturing steps that follow.

The fabrication file may utilize any recognizable solid model specification, such as ".amf" format [see ASTM WK27506 -New Specification for Data Exchange Format for Additive Manufacturing, available at http://www.astm.org/DATABASE.CART/WORKITEMS/WK27506.htm (accessed 07-29-2011)] or ".stl" format [see Standard Data Format for Fabbers, available at http://www.ennex.com/∼fabbers/StL.asp (accessed 07-29-2011)], and may be embodied on any sort of permanent storage medium (e.g., CD, CD-ROM, flash), semi-permanent (e.g., SRAM) or transitory (e.g., DRAM) storage medium, or embodied in a coded data signal.

Additional background details concerning the fabrication of medical devices using additive techniques can be found in: J. Vehvilainen, "Process and Web Application Development of Medical Applications of Additive Manufacturing," Master's Thesis, Aalto University, School of Science, Dec. 27, 2011 (available at http://lib.tkk.fi/Dipl/2011/urnl00592.pdf (accessed 06-13-12)).

Referring to FIG. 1A, an additional step can be inserted that adds outwardly-protruding "needles" on the outer surface(s) of the osteo-porous and/or osteo-derived coating(s). Such needles substantially increase the coefficient of friction of the coating. Having a high coefficient of friction is clinically advantageous because it provides stronger initial fixation, which is important before bone is able to grow onto/into the porous structure. Such needles can be uniformly or non-uniformly (as shown in FIG. 1B) distributed along the porous surface. Likewise, various shapes for the needles are possible, including rectangular (as depicted in FIG. 1B), pyramidal, conical, tube-shaped, etc. Also, the needles need not be oriented exactly normal to the exterior surface, but are preferably oriented in a substantially normal (e.g., within +/-15 degrees from normal) orientation. Furthermore, the orientation and/or shape of all needles need not be the same, and the needles may be rendered on selected portions, or the entirety, of the exterior coated surface(s).

Utilizing these or similar techniques, one can efficiently and advantageously form (and/or finish) implants of the sort depicted herein.

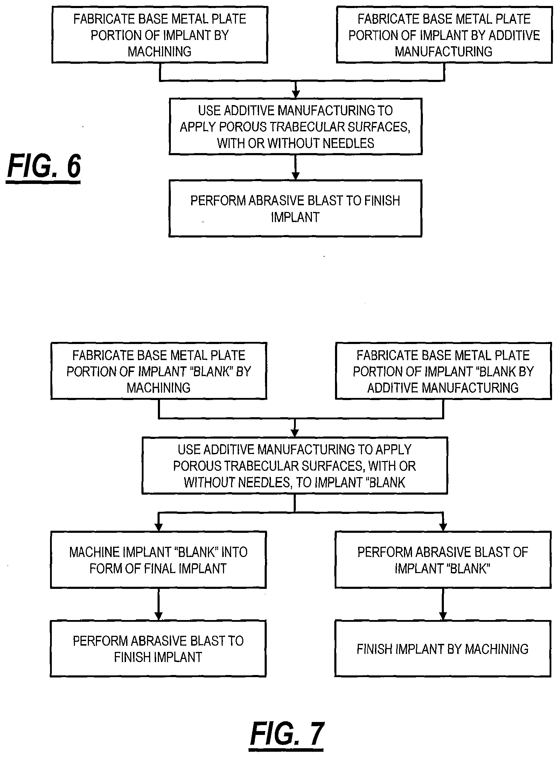

Referring to FIG. 6, two preferred process flows for fabricating and finishing implants using an abrasive blast are shown. FIG. 7 shows two alternative flows that employ the blasting technique as well. FIGS. 10A and 10B and 11A and 11B contrastingly depict SEM images of porous implant sections that have and have not undergone an abrasive blasting step. As can be seen from the images, the non-blasted samples are mostly smooth if viewed on the 20 or 100 µrn scale, whereas the blasted samples are almost entirely rough, when viewed at similar magnification. This blast process creates a texture approximately 1-20µm in scale. This is an ideal scale for microtexture because it helps osteoblasts adhere to the titanium struts. The bast material is not deposited on the strut (as an HA coating would be). It simply textures the titanium surface by erosion.

In accordance with the invention, the preferred abrasive blast process utilizes an MCD Apatitic Abrasive (Multi-phased Calcium Phosphate containing HA, TCP and other CaP phases) distributed by Hitemco Medical Applications, Inc., 160 Sweet Hollow Rd., Old Bethpage, NY 11804. The blast media has a 425 - 180 urn size range. The process utilizes a Comco AccuFlo^{®} standard tank micro-abrasive blaster, equipped with Simoom^{®} Technology and PowderGate^{®} valve. Tank orifice is 1.016 cm (0.40 inches); Nozzle is 0.1524 cm (0.060 inches); Pressure is 6.205 +/- 0.345 bar (90 +/- 5 psi). A satisfactory roughness has been achieved when the blast does not further affect the visual appearance, specifically the color and reflectivity of the device. Machined devices may require a blast touch up.

FIGS. 9A-9L illustratively depict the concept of slightly-irregular lattices that are ideally adapted for additive manufacturing. As shown in these figures, node perturbation refers to the location of intersecting struts. In accordance with this aspect of the invention, such intersection locations are be randomized such that the node deviates from a uniform lattice by a randomized distance. Strut size randomization refers to a deviation in cross-section dimension (e.g., strut diameter). Discrete struts in a lattice could have different cross-sectional sizes, or the struts could have a diameter gradient from one end to the other. These parameters can be randomized for greater diversity in the lattice's geometry. Such slightly-irregular lattices can be used to fabricate any sort of medical implant for which regular lattices are currently used, including, for example, those disclosed in the incorporated Pressacco et al. and Jones et al. applications.

Another illustrative application of the invention relates to bone fixation/fusion devices of the sort disclosed in U.S. Pat. No. 7,922,765 and/or U.S. Published Applic. No. 2011/0118841 that have been modified to include one or more needle-populated surface(s) in accordance with the teachings of the present invention.

Such fusion/fixation devices are preferably fabricated by additive techniques, may utilize multi-circular cross-sectional profiles (either uniform or tapered), and preferably include exterior needles, preferably oriented in a direction that would resist removal of the implant. Multi-circular cross-sectional profiles have the distinct advantage of not requiring one or more bore broaching steps, thus making insertion quicker and reducing patient infection risk.

Finally, it should be understood that the novel structures disclosed and enabled by the present invention are not limited exclusively to those manufactured using additive manufacturing. Indeed, as persons skilled in the art will appreciate, other known surface modification techniques - such as, for example, Surfi-Sculpt (U. S. Pat. No. 7,667,158) - may be used to produce the osteoporous, osteo-derived, and/or needle-containing textures of the inventive implants.

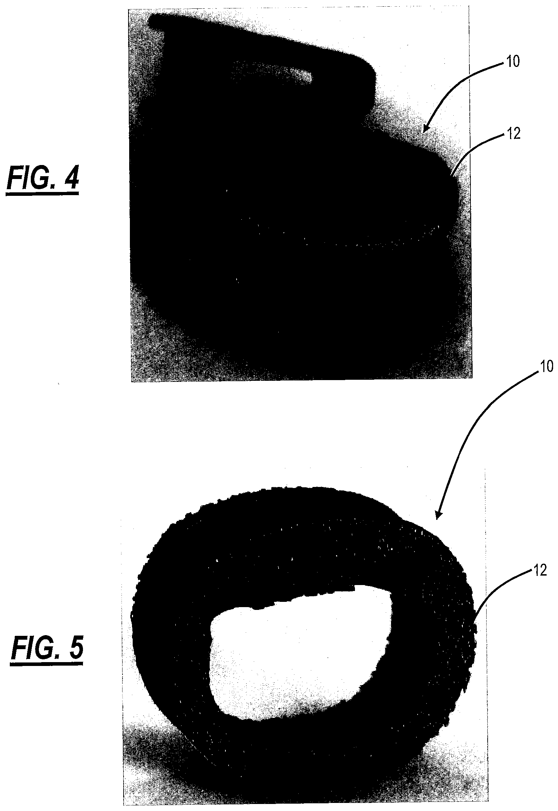

Referring now specifically to FIGS. 2-5, in one exemplary embodiment, the present invention provides an ALIF cage 10 for use in spinal surgical procedures. More specifically, the present invention provides an ALIF cage 10 that includes one or more porous surfaces 12 that promote bony fixation. Preferably, these porous surfaces 12 are formed via an additive manufacturing technique using a titanium alloy powder or the like (as is described in greater detail herein below), thereby providing a unitary structure and eliminating the need to use coatings or bondings for such purpose.

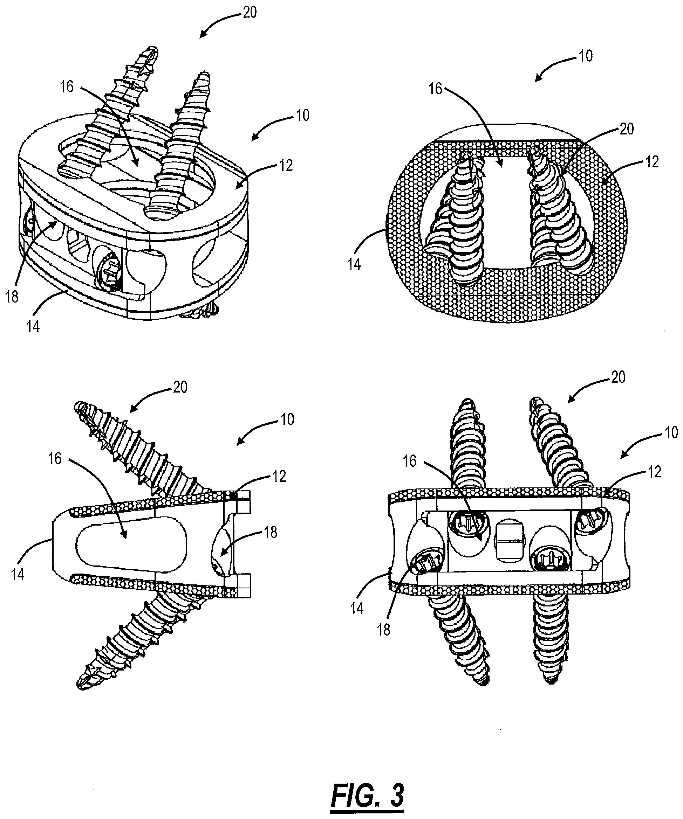

In general, the ALIF cage 10 includes a body structure 14 that defines a hollow interior portion 16 and a plurality of ports 18 through which screws 20 (FIG. 3) or the like are disposed, through the hollow interior portion 16 and into adjacent bone. Optionally, the hollow interior portion 16 is sized and configured to contain bone graft material or the like, to further enhance bony fixation. The screws 20 are shown at a given angle, although any suitable angle(s) for a given application may be utilized, as may any suitable number and size of screws 20.

Advantageously, the ALIF cage 10 consists of a unitary structure that is substantially solid in the middle, for example, and graduates to porous at given surfaces, for example. This provides enhanced structural integrity, allows for the porosity to be carefully controlled in a given area, etc. Alternatively, there may be a relatively sharp dividing line between the solid portion and the porous portions, although it is contemplated that they are integrally formed via the additive manufacturing technique.

The following description is partially taken from US 13/530,048 and explains how the porous surfaces 12 of the ALIF cage 10 are formed.

Generally speaking, and without intending to be limiting, one aspect of the present invention relates to improved medical implants that include, for example, at least the following: a primary structure formed from metal; and at least one needle-populated, metallic surface portion formed on at least one exterior portion of the primary structure, the at least one surface portion located such that it engages with a patient's bone when the implant is implanted in the patient. Such needle-populated, metallic surface portions may contain, for example, a collection of at least fifty, a hundred, two hundred, five-hundred or more needles, and may be further characterized by at least one, two, three, four, five or more of the following characteristics: (a) the needles in the collection are all oriented substantially normal to the surface portion; (b) the needles in the collection are all oriented in substantially the same direction, with the direction being other than normal to the surface portion; (c) the needles in the collection are all oriented in substantially the same direction, with the direction being other than normal to the surface portion, but within 15 degrees from the normal direction; (d) the needles in the collection are all oriented in substantially the same direction, with the direction being other than normal to the surface portion, and more than 15 degrees from the normal direction; direction (not claimed); (e) the collection includes needles oriented in at least three different directions relative to the surface portion; (f) the collection includes needles oriented in at least five different directions relative to the surface portion, with all of the needles oriented within 15 degrees from the surface portion normal direction; (g) all of the needles in the collection have substantially the same height; (h) the collection includes needles of at least three different heights; (i) all of the needles in the collection have substantially the same shape; (j) the collection includes needles of at least two different shapes; (k) the needles are distributed substantially uniformly over the surface portion; (1) the needles are distributed non-uniformly over the surface portion; (m) some of the needles in the collection are anchored to the primary structure; (n) most of the needles in the collection are anchored to the primary structure; (o) most of the needles in the collection are anchored to structural elements contained within an osteo-porous, osteo-derived or trabecular coating on the at least one exterior portion of the primary structure; and/or (p) all of the needles in the collection are anchored to structural elements contained within an osteo-porous, osteo-derived or trabecular coating on the at least one exterior portion of the primary structure. The at least one exterior portion preferably includes at least one osteo-porous surface, which may comprise at least one osteo-derived surface. The at least one osteo-porous surface and the needles may be simultaneously formed by an additive manufacturing process, such as, but not limited to, EBM or DMSLS. The primary structure may comprise, for example, the ALIF cage 10 of the present invention, a dental implant, a foot-and-ankle or long-bone osteotomy wedge, an intervertebral fusion device, a tibial/femoral augment or spacer, a tibial tray portion of a knee implant, a femoral component portion of a knee implant, a primary hip implant, a revision hip implant, a hip trauma component, an acetabular cup, a hip acetabular augment, or other appropriate structure.

Again, generally speaking, and without intending to be limiting, another aspect of the present invention relates to method(s) for making a medical implants with at least one osteo-porous surface by, for example: forming at least a portion of a primary structure of the implant; and forming at least one needle-populated, metallic surface portion on at least one exterior portion of the primary structure using an additive manufacturing technique, the at least one needle-populated surface portion located such that it engages with a patient's bone when the implant is implanted in the patient.

The exemplary flow starts with a spongy bone sample, which is micro CT scanned to obtain 3D scan data, which is then processed into solid model data representing an osteo-porous or osteo-derived texture. This texture data is then combined with data representing the overall implant geometry to create a fabrication file for use by either of the manufacturing steps that follow. The fabrication file may utilize any recognizable solid model specification, such as ".amf" format [see ASTM WK27506 - New Specification for Data Exchange Format for Additive Manufacturing, available at http://www.astm.org/DATABASE.CART/WORKITEMS/WK27506.htm (accessed 07-29-2011)] or ".stl" format [see Standard Data Format for Fabbers, available at http://www.ennex.com/~fabbers/StL.asp (accessed 07-29-2011)], and may be embodied on any sort of permanent storage medium (e.g., CD, CD-ROM, flash), semi-permanent (e.g., SRAM) or transitory (e.g., DRAM) storage medium, or embodied in a coded data signal.

Additional background details concerning the fabrication of medical devices using additive techniques can be found in: J. VehvilLinen, "Process and Web Application Development of Medical Applications of Additive Manufacturing," Master's Thesis, Aalto University, School of Science, Dec. 27, 2011 (available at http://lib.tkk.fi/Dipl/2011/urnl00592.pdf (accessed 06-13-12)).

An additional step can be inserted that adds outwardly-protruding "needles" on the outer surface(s) of the osteo-porous and/or osteo-derived coating(s). Such needles substantially increase the coefficient of friction of the coating. Having a high coefficient of friction is clinically advantageous because it provides stronger initial fixation, which is important before bone is able to grow onto/into the porous structure. Such needles can be uniformly or non-uniformly distributed along the porous surface. Likewise, various shapes for the needles are possible, including rectangular, pyramidal, conical, tube-shaped, etc. Also, the needles need not be oriented exactly normal to the exterior surface, but are preferably oriented in a substantially normal (e.g., within +/-15 degrees from normal) orientation. Furthermore, the orientation and/or shape of all needles need not be the same, and the needles may be rendered on selected portions, or the entirety, of the exterior coated surface(s).

Utilizing these or similar techniques, one can efficiently and advantageously form (and/or finish) implants. Finally, it should be understood that the novel structures disclosed and enabled by the present invention are not limited exclusively to those manufactured using additive manufacturing. Indeed, as persons skilled in the art will appreciate, other known surface modification techniques -- such as, for example, Surfi-Sculpt (U.S. Pat. No. 7,667,158) -- may be used to produce the osteoporous, osteo-derived, and/or needle-containing textures of the inventive implants.

FIGS. 12A and 12B are side and top planar views of one exemplary embodiment of the posterior lumbar interbody fusion (PLIF) cage 100 of the present invention. The PLIF cage 100 includes a body structure 102 that defines a substantially hollow interior portion 104 and incorporates one or more windows 106, such that graft material or the like may be disposed within the PLIF cage 100 to promote bony purchase. The PLIF cage 100 also includes a leading edge 108 that is substantially tapered, such that the PLIF cage 100 may be easily disposed in an intervertebral space, and a trailing edge that includes a tool receiving recess 110 or the like. As with all devices of the present invention, the PLIF cage 1001 may be manufactured from PEEK or a metallic material with the porous surface(s) described in detail herein.

FIGS. 13A-13C are top planar, side planar, and bullet views of one exemplary embodiment of the transforaminal lumbar interbody fusion (TLIF) cage 200 of the present invention. The TLIF cage 200 includes a substantially-curved body structure 202 that defines a substantially hollow interior portion 204 and incorporates one or more windows 206, such that graft material or the like may be disposed within the TLIF cage 200 to promote bony purchase. The TLIF cage 200 also includes a leading edge 208 that is substantially tapered, such that the TLIF cage 200 may be easily disposed in an intervertebral space, and a trailing edge that includes a tool receiving recess 210 or the like. As with all devices of the present invention, the TLIF cage 200 may be manufactured from PEEK or a metallic material with the porous surface(s) described in detail herein.

FIG. 14 is perspective, side planar, and top planar views of one exemplary embodiment of the oblique lumbar interbody fusion (OLIF) cage 300 of the present invention. The OLIF cage 300 includes a body structure 302 that defines a substantially hollow interior portion 304 and incorporates one or more windows 306, such that graft material or the like may be disposed within the OLIF cage 300 to promote bony purchase. The OLIF cage 300 also includes a leading edge 308 that is substantially tapered, such that the OLIF cage 300 may be easily disposed in an intervertebral space, and a trailing edge that includes a tool receiving recess 310 or the like. As with all devices of the present invention, the OLIF cage 300 may be manufactured from PEEK or a metallic material with the porous surface(s) described in detail herein.

FIG. 15 is perspective views of the cervical cages 350 of the present invention, both in PEEK and titanium embodiments, and incorporating a novel locking mechanism 400. These cervical cages 350 are similar to the ALIF cage described in detail herein above, further including the novel locking mechanism 400. The novel locking mechanism 400 includes a cover plate 402 that is held in place over one of the primary cage screws using a plate screw 404. When actuated, the plate screw 404 compresses the cover plate 402 against the body structure of the cervical cage 350, driving locking tabs 406 in the corners of the cover plate 402 into corresponding recesses manufactured into the body structure. This locks the primary cage screws into the body structure and prevents them from backing out. Advantageously, at least three points of contact are provided between the cover plate 402 and the body structure to provide the required stability.

FIG. 16 is top and side planar views of the cervical cage of the present invention, in the titanium embodiment and including at least one porous surface 420 formed by the process of FIG. 1. In this exemplary embodiment, the porous surface 420 does not cover the insertion edge and is thinner at the anterior edge to allow support material for the cover plate 402 and plate screw 404.

FIG. 17 is a series of perspective views illustrating the operation of the locking mechanism of FIG. 15.

FIG. 18 is a perspective view of the ALIF cage of the present invention, in a PEEK embodiment and including a novel locking mechanism. FIG. 19 is a perspective view of the ALIF cage of the present invention, in a PEEK embodiment and including a novel locking mechanism. These ALIF cages operates in a manner similar to the cervical cages described herein above.

FIG. 20 is a perspective view of the surgical screw 500 of the present invention, incorporating both porous regions 502 and non-porous regions 504. Specifically, in this exemplary embodiment, the porous regions are disposed at the threads to promote bony purchase.

## Claims

1. A surgical implant device, comprising:
a metallic body portion; and
one or more metallic surfaces comprising a metallic osteo-porous or osteo-derived layer and a plurality of needles on an exterior portion of the metallic body portion;
wherein the metallic body portion and the one or more metallic surfaces comprising the metallic osteo-porous or osteo-derived layer and the plurality of needles are integrally formed,
wherein the metallic osteo-porous or osteo-derived layer comprises an irregular lattice of intersecting struts that intersects at randomly perturbed nodes,
wherein some of the plurality of needles are anchored to the metallic body portion and some of the plurality of needles are anchored to the intersecting struts of the irregular lattice of the metallic osteo-porous or osteo-derived layer,
wherein the plurality of needles substantially increase the coefficient of friction of the one or more metallic surfaces.

2. The surgical implant device of claim 1, wherein the plurality of needles are disposed substantially perpendicular to the body portion.

3. The surgical implant device of claim 1, wherein the plurality of needles are disposed at an angle to the body portion within 15 degrees from the normal direction.

4. The surgical implant device of claim 1, wherein the plurality of needles comprise titanium.

5. The surgical implant device of claim 1, wherein the body portion defines a hollow interior cavity.

6. The surgical implant device of claim 1, wherein the body portion defines one or more ports that are configured to receive a bone screw.

7. The surgical implant device of claim 1, wherein the body portion defines one or more ports that are configured to allow bony ingrowth.

8. The surgical implant device of claim 1, wherein the surgical implant device comprises one of an anterior lumbar interbody fusion cage, a posterior lumbar interbody fusion cage, a transforaminal lumbar interbody fusion cage, an oblique lumbar interbody fusion cage, a cervical cage, and a bone screw.

## Patentansprüche

1. Chirurgische Implantatsvorrichtung, umfassend:
einen metallischen Körperabschnitt; und
eine oder mehrere metallische Flächen, die eine metallische knochenporige oder vom Knochen abgeleitete Schicht umfassen; und eine Vielzahl von Nadeln auf einem Außenteil des metallischen Körperabschnitts;
wobei der metallische Körperabschnitt und die einen oder mehreren metallischen Flächen, die die metallische knochenporige oder vom Knochen abgeleitete Schicht umfassen, und die Vielzahl von Nadeln einstückig ausgeformt sind,
wobei die metallische knochenporige oder vom Knochen abgeleitete Schicht ein unregelmäßiges Gitter sich schneidender Streben, die einander an zufällig gestörten Knoten schneiden, umfasst,
wobei ein Teil der Vielzahl von Nadeln an dem metallischen Körperabschnitt verankert sind und ein Teil der Vielzahl von Nadeln an den sich schneidenden Streben des unregelmäßigen Gitters der metallischen knochenporigen oder vom Knochen abgeleiteten Schicht verankert sind,
wobei die Vielzahl von Nadeln den Reibungskoeffizienten der einen oder mehreren metallischen Flächen wesentlich erhöhen.

2. Chirurgische Implantatsvorrichtung gemäß Anspruch 1, wobei die Vielzahl von Nadeln im Wesentlichen senkrecht zu dem Körperabschnitt angeordnet sind.

3. Chirurgische Implantatsvorrichtung gemäß Anspruch 1, wobei die Vielzahl von Nadeln unter einem Winkel von höchstens 15 Grad gegenüber der Richtung der Normalen zu dem Körperabschnitt angeordnet sind.

4. Chirurgische Implantatsvorrichtung gemäß Anspruch 1, wobei die Vielzahl von Nadeln Titan umfasst.

5. Chirurgische Implantatsvorrichtung gemäß Anspruch 1, wobei der Körperabschnitt einen hohlen Innenraum definiert.

6. Chirurgische Implantatsvorrichtung gemäß Anspruch 1, wobei der Körperabschnitt einen oder mehrere Anschlüsse definiert, die so konfiguriert sind, dass sie eine Knochenschraube aufnehmen können.

7. Chirurgische Implantatsvorrichtung gemäß Anspruch 1, wobei der Körperabschnitt einen oder mehrere Anschlüsse definiert, die so konfiguriert sind, dass sie das Einwachsen des Knochens ermöglichen können.

8. Chirurgische Implantatsvorrichtung gemäß Anspruch 1, wobei die chirurgische Implantatsvorrichtung eines aus einem anterioren lumbalen interkorporellen Fusionskäfig, einem posterioren lumbalen interkorporellen Fusionskäfig, einem transforaminalen lumbalen interkorporellen Fusionskäfig, einem schrägen lumbalen interkorporellen Fusionskäfig, einem zervikalen Käfig und einer Knochenschraube umfasst.

## Revendications

1. Dispositif pour implant chirurgique, comprenant :
une partie de corps métallique, et
une ou plusieurs surfaces métalliques comprenant une couche métallique ostéo-poreuse ou ostéo-dérivée, et une pluralité d'aiguilles sur une partie extérieure de ladite partie de corps métallique,
dans lequel ladite partie de corps métallique et lesdites une ou plusieurs surfaces métalliques comprenant la couche métallique ostéo-poreuse ou ostéo-dérivée, et ladite pluralité d'aiguilles sont formées intégralement,
dans lequel ladite couche métallique ostéo-poreuse ou ostéo-dérivée comprend un réseau irrégulier d'entretoises s'entrecoupantes qui entrecoupe à des nœuds perturbés aléatoirement,
dans lequel une partie de ladite pluralité d'aiguilles sont ancrées dans la partie de corps métallique, et une partie de ladite pluralité d'aiguilles sont ancrées dans lesdites entretoises entrecoupantes dudit réseau irrégulier de la couche métallique ostéo-poreuse ou ostéo-dérivée,
dans lequel la pluralité des aiguilles augmentent sensiblement le coefficient de frottement desdites une ou plusieurs surfaces métalliques.

2. Dispositif pour implant chirurgique selon la revendication 1, dans lequel ladite pluralité d'aiguilles sont disposées sensiblement perpendiculaire à la partie de corps.

3. Dispositif pour implant chirurgique selon la revendication 1, dans lequel ladite pluralité d'aiguilles sont disposées sous un angle maximal de 15 degrés de la direction normale par rapport à la partie de corps.

4. Dispositif pour implant chirurgique selon la revendication 1, dans lequel ladite pluralité d'aiguilles comprennent du titane.

5. Dispositif pour implant chirurgique selon la revendication 1, dans lequel ladite partie de corps définit une cavité intérieure creuse.

6. Dispositif pour implant chirurgique selon la revendication 1, dans lequel ladite partie de corps définit un ou plusieurs raccords qui sont configurés pour recevoir une vis à os.

7. Dispositif pour implant chirurgique selon la revendication 1, dans lequel ladite partie de corps définit un ou plusieurs raccords qui sont configurés pour permettre la croissance osseuse dans l'implant.

8. Dispositif pour implant chirurgique selon la revendication 1, dans lequel ledit dispositif pour implant chirurgique comprend l'une parmi une cage à fusion intervertébrale antérieure lombaire, une cage à fusion intervertébrale postérieure lombaire, une cage à fusion intervertébrale lombaire transforaminale, une cage à fusion intervertébrale lombaire oblique, et une vis à os.
